# FASCICULE DE BREVET EUROPEEN

(11) **EP 1 317 931 B1**
(45) Date de publication et mention de la délivrance du brevet: **07.04.2004**
(21) Numéro de dépôt: 02080130.4
(22) Date de dépôt: 02.12.2002
(51) Int. Cl.: A61L 2/07, A61L 11/00

(54) **Procédé de traitement d'effluents liquides et installation prévue à cet effet**
Verfahren und Vorrichtung zur Behandlung von flüssigen Abfällen
Process and apparatus for treating liquid effluents

(30) Priorité: 07.12.2001 BE 200100796
(43) Date de publication de la demande: 11.06.2003
(73) Titulaire: Chonquerez, Alain, 1070 Bruxelles (BE)
(72) Inventeur: Chonquerez, Alain, 1070 Bruxelles (BE)
(74) Mandataire: Ostyn, Frans

(56) Documents cités:
- DE-A- 1 805 985
- DE-A- 1 933 542
- DE-C- 19 827 404
- FR-A- 1 325 257
- US-A- 5 223 229

## Description

L'invention concerne le traitement de déchets (effluents) liquides présentant un risque de contamination biologique pour l'environnement et plus particulièrement de traitement thermique de tels effluents. La contamination biologique peut provenir d'activités de recherche ou de production à caractère médical our pharmaceutique. La contamination peut être d'origine microbienne (modifiée génétiquement ou non), biologique par mutation, de culture cellulaire ou toutes combinaisons de celles-ci. La présente invention se rapporte à un procédé de traitement thermique par lot (appelé cycle de décontamination ou décontamination, de déchets liquides provenant de laboratoires ou locaux de production à caractère médical ou pharmaceutique en garantissant la sécurité de non-contamination de l'environnement et des opérateurs.

La stérilisation est le processus de destruction des microorganismes qui conduit à l'absence de croissance d'un échantillon dans un milieu de culture approprié. Le processus habituellement appliqué est une exposition à 121°C ou 134°C (-0; +3°C) pendant 15 à 60 minutes, temps de chauffage et refroidissement non compris. Dans tous les cas, l'effet thermique recherché ne peut être obtenu avec toute garantie que lorsqu'une homogénéité de température dans l'ensemble de la cuve de traitement du liquide peut être démontrée aussi bien dans le liquide que dans tout partie de l'enceinte jusqu'aux vannes d'arrêts, en ce compris la partie supérieure non immergée. Un critère d'acceptation de l'homogénéité de température est que l'écart de température entre le point le plus froid et le plus chaud de l'enceinte n'excède pas 0.5°C pendant la phase de décontamination.
On connaît des installations de traitement d'effluents biologiques en continu ou par lot, mais toutes présentent l'inconvénient de ne pas permettre d'obtenir les critères de validation décrits ci-dessus et de donner une sécurité biologique à l'égard de l'environnement. Les critères de validation ne sont en général pas atteints par suite d'une mauvaise évacuation de l'air, ce qui provoque la présence de "points froids" pendant la phase de décontamination. Cette mauvaise évacuation de l'air est due au concept de la cuve qui présente dans la partie supérieure de nombreuses connections dans lesquelles l'air reste emprisonné.
Les risques à l'égard de l'environnement sont liés au fait que la conception des systèmes ne tient pas compte des situations anormales de fonctionnement en cas de défaillance ou de nécessité d'intervention technique. L'environnement peut-être contaminé, par exemple, par suite de l'ouverture d'une soupape de sûreté ou par la nécessité de démonter une pompe non stérilisée.

Installations pour le traitement d'effluents liquides sont connues par FR-A-1325257 et DE-C-19827404.

La présente invention vise à remédier à l'ensemble des inconvénients précités, par une approche globale de tout le processus de fonctionnement tout en éliminant les risques biologiques liés à la manipulation des effluents contaminés.

Pour ce faire, le procédé et l'installation selon l'invention comprennent notamment une enceinte isolée de l'environnement par un système de filtration stérilisante destinée à la récolte des effluents contaminés et une cuve sous pression pour le traitement thermique des effluents. L'installation est reliée aux accessoires nécessaires à l'injection de vapeur, aux moyens de mise sous vide et au nettoyage, et peut être conçue pour le traitement de différentes capacités.

L'invention concerne ainsi, en particulier, un procédé de traitement d'effluents liquides par stérilisation des effluents dans un autoclave, dans lequel les gaz présents dans l'autoclave et/ou dissous dans les effluents à traiter situés dans l'autoclave, sont évacués de celui-ci.
Pour ce faire, un vide est appliqué sur la partie supérieure de la cuve à l'aide d'une pompe à vide.

Selon la réalisation préférée de l'invention, les moyens d'évacuation des gaz sont maintenus en dehors des circuits contaminés par les effluents à traiter. Ces moyens, et plus particulièrement la pompe à vide n'est alors pas dans le circuit contaminé. Elle peut être remplacée en cas de défaillance technique sans risque de contamination biologique de l'environnement ou de l'opérateur.

En appliquant ce mode de réalisation les odeurs éventuellement émises par les effluents sont mélangées à l'eau de la pompe à vide et éliminées par les égouts. De cette manière il n'y a pas d'inconfort olfactif dû au fonctionnement de la station de décontamination.

Les gaz présents dans l'autoclave et/ou dissous dans les effluents à traiter sont de préférence évacués à l'aide d'une pompe à vide, un filtre d'une porosité inférieure à 0,2 µm étant disposé entre l'autoclave et la pompe à vide.
On préconise en particulier un filtre absolu d'une porosité maximale de 0.2µm installé sur la ligne d'aspiration entre l'autoclave et la pompe à vide afin de retenir les micro-organismes qui seraient éventuellement aspirés.

Selon une particularité de l'invention, les effluents à traiter peuvent être dégazés par le chauffage, par injection directe, de vapeur dans le liquide situé dans l'autoclave, par exemple par injection de vapeur au travers d'un injecteur spécial répartissant la vapeur dans la masse d'effluents.
Dans ce mode de réalisation de l'invention, on utilise de préférence une pompe à anneau liquide, afin d'aspirer, sans dommage pour la pompe, des vapeurs chaudes. Le refroidissement de la pompe est fait en eau perdue.

Selon une autre particularité de l'invention, il est préférable de maintenir l'aspiration des gaz pendant toute la durée du chauffage. Plus particulièrement la pression est maintenue à une valeur de consigne jusqu'à l'obtention d'une température de 99°C. Ensuite, la pression et la température montent progressivement jusqu'à la température de décontamination en suivant la courbe de saturation de vapeur (courbe de Mollier). L'homogénéité de température est démontrée par la technique de validation consistant à disposer dans l'enceinte de la cuve des sondes de température reliées à un système d'acquisition de données. Les températures sont enregistrées à intervalle régulier (par exemple toutes les 30 secondes) durant tout le processus de décontamination.

Une autre particularité préférée du procédé selon l'invention consiste à veiller à ce que la pression de la vapeur injectée soit à tout instant supérieure à la pression dans l'autoclave, afin d'éliminer tout risque de contamination par le contenu de l'autoclave.

Dans ce mode de réalisation du procédé selon l'invention, l'alimentation de vapeur est de préférence protégée par des soupapes de sûreté, tandis que l'autoclave est dépourvu de soupapes de sûreté le risque de contamination biologique en cas de surpression est ainsi éliminé.

Selon encore une autre particularité de l'invention, le volume de l'autoclave est de préférence prévu de manière à permettre l'accumulation de condensats de vapeur en plus du volume des effluents à traiter.

Selon le procédé de l'invention, le filtre absolu est utilisé de préférence stérilisable à la vapeur, afin de détruire les micro-organismes qui pourraient s'accumuler sur la membrane de filtration.

Dans ce cas, le procédé implique de préférence un cycle spécial de stérilisation du filtre avec contrôle de la température dans le filtre.

Selon l'invention, on peut en particulier appliquer la technique du vide au transfert du volume d'effluents à traiter vers l'autoclave. Ce transfert peut être contrôlé soit par pesée de l'autoclave, soit par une sonde de niveau placée dans l'autoclave. Les deux systèmes peuvent être utilisés en système redondant (double contrôle) pour des raisons de sécurité.
Le refroidissement de la charge en fin de cycle de décontamination peut notamment se faire par circulation externe à l'autoclave sur un échangeur de chaleur alimenté en eau de refroidissement.

Selon encore une particularité de l'invention, le déroulement des opérations est de préférence géré par un logiciel contrôlant les paramètres de fonctionnement et/ou générant des alarmes sonores et/ou visuelles en cas de défaillances.
Dans ce cas les alarmes sont classifiées selon un degré de criticité. Les alarmes non critiques sont signalées mais n'interrompent pas le processus. Si une alarme critique survient, le processus de décontamination est interrompu et la station de décontamination est placée dans un mode de sécurité qui empêche toute contamination de l'environnment.
Il peut, en outre, être prévu que des systèmes de commandes manuelles permettent de pallier à une défaillance de l'un ou plusieurs éléments du pilotage automatique afin de pouvoir intervenir ultérieurement sur le système défaillant avec toutes les garanties d'absence de risque biologique.

Selon un mode de réalisation particulier de l'invention, le volume de l'autoclave peut notamment être prévu de manière à permettre au moins deux cycles de stérilisation, de préférence au moins trois cycles de stérilisation, avant la décharge.

L'invention concerne également une installation de traitement d'effluents liquides par stérilisation des effluents dans un autoclave, comprenant notamment des moyens d'évacuation des gaz présents dans l'autoclave et/ou dissous dans les effluents à traiter situés dans l'autoclave.

Selon un mode de réalisation préféré de l'installation, les moyens d'évacuation des gaz sont situés en dehors des circuits contaminés.
Ces moyens comprennent, de préférence, une pompe à vide, un filtre d'une porosité inférieure a 0,2 µm étant de préférence disposé entre l'autoclave et la pompe à vide.

Selon une particularité supplémentaire de l'invention, l'installation peut, en outre, comprendre des moyens d'alimentation de vapeur vers l'autoclave, protégés par des soupapes de sécurité, tandis que l'autoclave lui-même est dépourvu de soupapes de sécurité.

Dans ce cas, la pompe à vide est, de préférence, du type à anneau liquide, afin d'aspirer, sans dommage pour la pompe, des vapeurs chaudes. Le refroidissement de la pompe est fait en eau perdue.

Selon encore une autre particularité de l'installation selon l'invention le volume de l'autoclave est de préférence suffisant pour permettre au moins deux cycles de stérilisation, plus particulièrement au moins trois cycles de stérilisation, avant la décharge des effluents traités.

Selon encore un mode de réalisation de l'invention, l'autoclave de l'installation peut, en outre, comprendre des moyens de détection du volume de liquide dans l'autoclave, fonctionnant de préférence par pesée et/ou par sonde(s) de niveau.

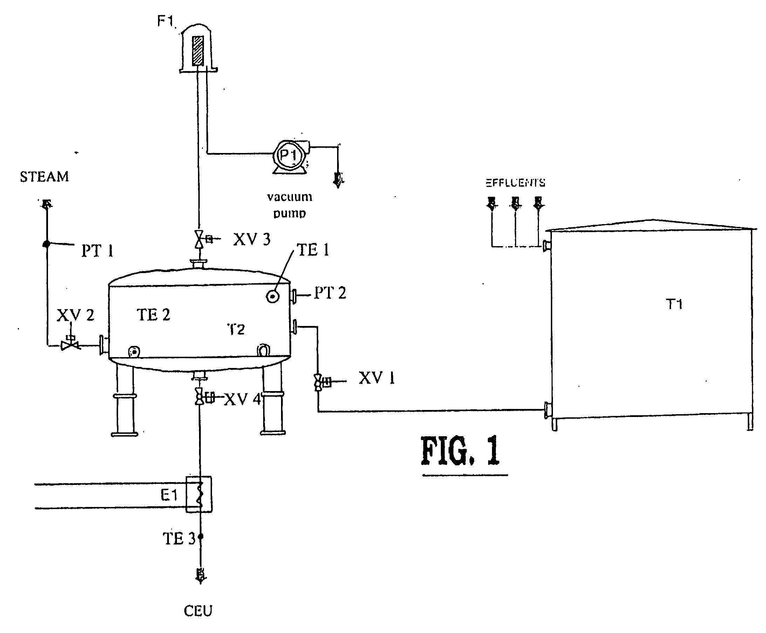

D'autres particularités de l'invention, ainsi que sa mise en oeuvre, apparaîtront dans la description qui suit d'un mode de réalisation particulier donné à titre d'exemple purement illustratif en se référant à la figure annexée repésentant schématiquement une installation selon l'invention.
Il est à noter que les aspects spécifiques de cet exemple ne limitent en rien la portée de l'invention telle que décrite ci-dessus et telle que définie dans les revendications qui suivent.

L'installation représentée sur la figure comprend une cuve tampon T1, un autoclave T2, une pompe à vide P1, un filtre F1 et un échangeur E1 , ainsi que des vannes XV1 - XV4, des détecteurs de pression PT1 - PT2 et des détecteurs de température TE1 - TE3.

Le processus opère selon le schéma de principe suivant :
Le système collecte continuellement les eaux des égouts dans un réservoir de collecte (cuve tampon T1). Quand un niveau minimal est atteint dans le réservoir tampon, le liquide est transféré à un autoclave T2 de stérilisation jusqu'à un niveau prédéterminé.
La stérilisation de l'effluent est exécutée selon le principe « du traitement de matériel hautement pathogène ».
Le mélange de l'effluent pour une distribution de chaleur uniforme dans la charge est exécuté par l'injection directe de vapeur au fond de l'autoclave.
Après le contrôle et l'acceptation des paramètres de stérilisation par le contrôleur de processus, les effluents sont refroidis à une température acceptable pour la décharge au collecteur d'égout. Une procédure de contrôle du système maintiendra l'effluent si l'autoclave de stérilisation n'a pas terminé le cycle. La stérilisation par la vapeur peut être exécutée manuellement en cas d'échec du processus automatique.

Le procédé implique les trois étapes générales suivantes :

### Collecte des effluents

Les effluents provenant de différentes zones de travail sont rassemblés dans le réservoir tampon (T1). Le système travaille sur la base d'un processus continu de collecte et le transfert séquentiel à l'autoclave.

### Stérilisation de l'autoclave

L'autoclave est mis sous vide au moyen de la pompe à vide - VACUUM PUMP - (P1) au travers du filtre (F1). La pression résiduelle est maintenue pendant toute la phase de chauffage.
Lorsque le transfert est terminé, la vanne de fond se ferme et l'injection directe de vapeur commence le chauffage des effluents. La température de stérilisation est maintenue durant le temps requis.

### Drainage

Quand la commande de processus a vérifié la conformité des paramètres de stérilisation, les effluents sont évacués par un collecteur et refroidis à la température exigée

Ces trois étapes principales suivent le processus détaillé suivant :

### Pré-requis de la décontamination

Trois paramètres doivent être déterminés selon une procédure de validation :
Température minimale de décontamination
Température de consigne de décontamination
Temps de décontamination
Remplissage de l'autoclave

Aussitôt que le niveau minimum de remplissage de la cuve tampon (T1) est atteint, XV1 s'ouvre. Le liquide est transféré jusqu'à un niveau décidé, contrôlé par le contrôleur de niveau ou le contrôleur pondéral de l'autoclave.
Le transfert s'arrête immédiatement si niveau bas/bas dans T1 est atteint pendant le transfert pour éviter la succion d'air dans l'autoclave. Le niveau dans T1 doit à nouveau atteindre le niveau minimal pour poursuivre le remplissage de l'autoclave. Quant l'autoclave est rempli au niveau choisi, la phase suivante (le chauffage) commencera après fermeture de XV1.

### Alarmes de phase de remplissage

Pendant le cycle si le controle de niveau dans le réservoir tampon est bas/bas la condition d'alarme est localement indiquée. Si la cellule de niveau détecte un niveau haut dans l'autoclave (T2), toutes les opérations sont interrompues, une alarme est localement indiquée et une alarme générale « station de décontamination » est annoncée au réseau de contrôle d'alarme d'usine.
Si une pression incorrecte est détectée dans l'autoclave par PT2, toutes les opérations sont interrompues, une alarme est localement indiquée et une alarme générale « station de décontamination » est annoncée au réseau de contrôle d'alarme d'usine. Le cycle de stérilisation en cours est interrompu. L'autoclave est isolé de l'environnement en fermant toutes les vannes. Une alarme peut aussi avoir lieu si le bouton d'arrêt de secours est activé.

### Phase de chauffage

XV3 est ouvert. La pompe à vide P1 réduira à nouveau la pression à une valeur pré-choisie. (Par exemple 0.1 bar).
Quand le vide est atteint, la vapeur à 3 barg (STEAM) sera injectée directement dans l'autoclave par XV2. La pompe à vide maintiendra la pression (mesuré par PT2) aussi bas que possible jusqu'à ce que la température mesurée par TE 1 atteint 100°C. La vannes XV3 se fermera alors.

La pression augmentera alors graduellement pendant l'augmentation de la température. Quand TE1 et TE2 indiquent la température du point de réglage (par exemple 125°C), le temporisateur de phase de décontamination commence.

### Alarmes de phase de chauffage

Si la pression de vapeur, mesurée par PT2, tombe en-dessous de 2 barg, l'injection de la vapeur s'arrête, toutes les vannes se ferment pour isoler l'autoclave de l'environnement, une alarme est localement indiquée et une alarme générale « station de décontamination » est annoncée au réseau de contrôle d'alarme d'usine.
Si la température mesurée TE1 et TE2 n'a pas atteint la température de point de réglage après que le temporisateur de phase soit écoulé, une alarme est localement indiquée et une alarme générale « station de décontamination » est annoncée au réseau de contrôle d'alarme d'usine.
Une alarme peut aussi avoir lieu si le bouton d'arrêt de secours est activé.

### Décontamination

Les effluents sont maintenus à la température de point de réglage pendant le temps pré-réglé.

### (voir Pré-requis)

Si la température chute au-dessous de ce point de réglage, la vapeur est injectée par la vanne XV2. Quand le temps est écoulé, la phase de refroidissement/drainage commence.

### Alarmes de phase de décontamination

Si la température chute au-dessous de la température minimum pendant cette phase, le temporisateur de phase est interrompu sans remise à zéro.
Le temporisateur est relancé seulement une fois que la température à la sonde TE1 et TE2 est revenu à la température de point de réglage.
Si la température reste plus de 15 minutes en-dessous du point de réglage, une alarme est localement indiquée et une alarme générale « station de décontamination» est annoncée au réseau de contrôle d'alarme d'usine.
Si on observe une erreur de niveau persistante ou une trop haute pression pendant cette phase, l'autoclave est isolé immédiatement de l'environnement (toutes les vannes se ferment) et le temporisateur est interrompu. Une alarme est localement indiquée et une alarme générale « station de décontamination » est annoncée au réseau de contrôle d'alarme d'usine.
Une alarme peut aussi avoir lieu si le bouton d'arrêt de secours est activé.

### Phase de refroidissement/drainage

La vanne XV4 s'ouvre, les effluents sont refroidis dans le collecteur de drain-échangeur (El) par addition d'eau froide et évacués en CEU. Quand l'autoclave est vide, un nouveau cycle peut être commencé.

### Alarmes phase de refroidissement/drainage

Si la sonde de température TE3 indique une température persistante trop élevée, l'autoclave est isolé immédiatement de l'environnement (toutes les vannes sont fermées. Une alarme est localement indiquée et une alarme générale «station de décontamination » est annoncée au réseau de contrôle d'alarme d'usine.

### Stérilisation du filtre

La stérilisation du filtre est recommandée avant une opération de maintenance afin de protéger le personnel d'un risque biologique éventuel.

### Urgences et situations anormales

### Défaut du contrôleur de processus

En cas de panne du contrôleur de processus, il est possible de contrôler manuellement les activateurs pneumatiques des vannes pour exécuter les exécutions nécessaires.

### Alarmes

Le panneau de commande est équipé d'un affichage sur lequel un texte décrivant la cause de l'alarme est lisible et facilement compréhensible par l'utilisateur.
Le cas échéant, une alarme générale est envoyée sur le réseau d'usine.

## Revendications

1. Procédé de traitement d'effluents liquides par stérilisation des effluents dans un autoclave, **caractérisé en ce que** le remplissage de l'autoclave est effectué par application de vide sur l'autoclave.

2. Procédé selon la revendication 1, **caractérisé en ce que** l'air et/ou les autres gaz présents et/ou dissous dans les effluents à traiter situés dans l'autoclave, sont évacués de l'autoclave.

3. Procédé selon la revendication 2, **caractérisé en ce que** l'air et/ou les autres gaz sont évacués à l'aide d'une pompe à vide à anneau d'eau au travers d'un filtre stérilisant l'air et les gaz, ledit filtre étant stérilisable en place dans son carter.

4. Procédé selon l'une ou l'autre des revendications précédentes, **caractérisé en ce que** les effluents à traiter sont dégazés par injection directe de vapeur dans les effluents situés dans l'autoclave jusqu'à ce que les effluents à traiter atteignent une température supérieure à 99°C et **en ce qu'**ensuite on interrompt l'application de vide sur l'autoclave et on laisse monter la température (et la pression) jusqu'à une valeur sélectionnée de décontamination/stérilisation.

5. Procédé selon l'une ou l'autre des revendications 2 à 4, **caractérisé en ce que** les moyens d'évacuation de l'air et des gaz sont maintenus en dehors des circuits contaminés par les effluents.

6. Installation de traitement d'effluents liquides par stérilisation des effluents dans un autoclave, **caractérisé en ce qu'**elle comprend des moyens pour appliquer un vide sur l'autoclave, permettant le transfert des effluents liquides dans l'autoclave.

7. Installation selon la revendication 6, **caractérisée en ce qu'**elle comprend des moyens d'évacuation de l'air et/ou les autres gaz présents et/ou dissous dans les effluents à traiter situés dans l'autoclave.

8. Installation selon l'une ou l'autre des revendication 6 et 7, **caractérisée en ce qu'**elle comprend au moins une pompe à vide à anneau liquide d'eau et au moins un filtre stérilisant, destinés à transférer les effluents dans l'autoclave.

9. Installation selon l'une ou l'autre des revendications 7 et 8, **caractérisée en ce que** les moyens d'évacuation de l'air et des gaz sont situés en dehors des circuits contaminés par les effluents.

10. Installation selon l'une ou l'autre des revendication 7 à 9, **caractérisée en ce qu'**elle comprend des moyens d'alimentation de vapeur dans les liquides situés dans l'autoclave et des moyens d'alimentation de vapeur vers l'autoclave comprennent des soupapes de sécurité, tandis que l'autoclave lui-même en est dépourvu.

## Claims

1. A process for treating liquid effluents by sterilization of the effluents in an autoclave, **characterized in that** the filling of the autoclave is performed by application of a vacuum to the autoclave.

2. The process according to claim 1, **characterized in that** the air and/or other gases present and/or dissolved in the effluents to be treated in the autoclave are evacuated from the autoclave.

3. The process according to claim 2, **characterized in that** the air and/or other gases are evacuated using a water ring vacuum pump through a filter sterilizing the air and the gases, which filter being sterilizable in-situ in its housing.

4. The process according to any of the previous claims, **characterized in that** the effluents to be treated are degassed by direct injection of steam into the effluents in the autoclave until the effluents to be treated reach a temperature higher than 99 °C, and **in that** the creation of a vacuum in the autoclave is then interrupted to allow the temperature (and the pressure) to rise to a preselected decontamination/sterilization value.

5. The process according to any of claims 2 to 4, **characterized in that** the means for evacuating the air and gases are kept outside the piping circuits contaminated by the effluents.

6. A facility for treating liquid effluents by sterilization of effluents in an autoclave, **characterized in that** it includes a means to create a vacuum in the autoclave, permitting the transfer of liquid effluents to the autoclave.

7. The facility according to claim 6, **characterized in that** it includes means for evacuating the air and/or other gases present and/or dissolved in the effluents to be treated in the autoclave.

8. The facility according to either of claims 6 or 7, **characterized in that** it includes at least one liquid water ring vacuum pump and at least one sterilizing filter for the purpose or transferring effluents to the autoclave.

9. The facility according to either of claims 7 or 8, **characterized in that** the means for evacuating the air and gases are located outside the circuits contaminated by effluents.

10. The facility according to any of claims 7 to 9, **characterized in that** it includes means to feed steam into the liquids in the autoclave and means to feed steam to the autoclave, including safety valves, whereas the autoclave itself is equipped with none.

## Patentansprüche

1. Verfahren zur Behandlung von flüssigen Abwässern durch Sterilisation der Abwässer in einem Autoklaven, **dadurch gekennzeichnet, dass** der Autoklav durch Anlegen von Vakuum am Autoklaven befüllt wird.

2. Verfahren nach Anspruch 1, **dadurch gekennzeichnet, dass** die Luft und/oder die anderen Gase, die vorliegen und/oder in den zu behandelnden, im Autoklaven befindlichen Abwässern gelöst sind, aus dem Autoklaven abgezogen werden.

3. Verfahren nach Anspruch 2, **dadurch gekennzeichnet, dass** die Luft und/oder die anderen Gase mittels einer Wasserring-Vakuumpumpe durch einen Filter abgezogen werden, der die Luft und die Gase sterilisiert, wobei dieser Filter direkt in seinem Gehäuse sterilisiert werden kann.

4. Verfahren nach einem der vorangehenden Ansprüche, **dadurch gekennzeichnet, dass** die zu behandelnden Abwässer durch direktes Einleiten von Dampf in die im Autoklaven befindlichen Abwässer entgast werden, bis die zu behandelnden Abwässer eine Temperatur von über 99 °C erreichen, und dadurch, dass anschließend das Anlegen des Vakuums an den Autoklaven unterbrochen wird und man die Temperatur (und den Druck) bis zu einem gewählten Dekontaminations-/Sterilisationswert ansteigen lässt.

5. Verfahren nach einem der Ansprüche 2-4, **dadurch gekennzeichnet, dass** die Mittel zum Abzug der Luft und der Gase außerhalb der durch die Abwässer kontaminierten Kreisläufe gehalten werden.

6. Vorrichtung zur Behandlung von flüssigen Abfällen durch Sterilisation der Abwässer in einem Autoklaven, **da durch gekennzeichnet, dass** diese Mittel zum Anlegen eines Vakuums an den Autoklaven umfasst, die den Transfer der flüssigen Abfälle in den Autoklaven ermöglichen.

7. Vorrichtung nach Anspruch 6, **dadurch gekennzeichnet, dass** diese Mittel zum Abziehen der Luft und/oder der anderen vorliegenden und/oder in den zu behandelnden, im Autoklaven befindlichen Abwässern gelösten Gase umfasst.

8. Vorrichtung nach einem der Ansprüche 6 und 7, **dadurch gekennzeichnet, dass** diese mindestens eine Wasserring-Vakuumpumpe und mindestens einen sterilisierenden Filter umfasst, die für den Transfer der Abwässer in den Autoklaven bestimmt sind.

9. Vorrichtung nach einem der Ansprüche 7 und 8, **dadurch gekennzeichnet, dass** die Mittel zum Abziehen der Luft und der Gase sich außerhalb der durch die Abwässer kontaminierten Kreisläufe befinden.

10. Vorrichtung nach einem der Ansprüche 7 bis 9, **dadurch gekennzeichnet, dass** diese Mittel zur Dampfzufuhr in die im Autoklaven befindlichen Flüssigkeiten umfasst und die Mittel zur Dampfzufuhr in den Autoklaven Sicherheitsventile umfassen, während der Autoklav selbst keine aufweist.
